(19) Europäisches Patentamt
European Patent Office
Office européen des brevets

(11) EP 2 041 336 B1

(12) **EUROPÄISCHE PATENTSCHRIFT**

(45) Veröffentlichungstag und Bekanntmachung des Hinweises auf die Patenterteilung:
**29.12.2010 Patentblatt 2010/52**

(21) Anmeldenummer: **07786853.7**

(22) Anmeldetag: **27.06.2007**

(51) Int Cl.:
***C25B 3/04*** *(2006.01)*

(86) Internationale Anmeldenummer:
**PCT/EP2007/056390**

(87) Internationale Veröffentlichungsnummer:
**WO 2008/003620 (10.01.2008 Gazette 2008/02)**

# (54) ELEKTROCHEMISCHE HERSTELLUNG STERISCH GEHINDERTER AMINE

ELECTROCHEMICAL PRODUCTION OF STERICALLY HINDERED AMINES

FABRICATION ÉLECTROCHIMIQUE D'AMINES À ENCOMBREMENT STÉRIQUE

(84) Benannte Vertragsstaaten:
**AT BE BG CH CY CZ DE DK EE ES FI FR GB GR HU IE IS IT LI LT LU LV MC MT NL PL PT RO SE SI SK TR**

(30) Priorität: **04.07.2006 EP 06116551**

(43) Veröffentlichungstag der Anmeldung:
**01.04.2009 Patentblatt 2009/14**

(73) Patentinhaber: **BASF SE**
**67056 Ludwigshafen (DE)**

(72) Erfinder:
- **GRIESBACH, Ulrich**
  **68305 Mannheim (DE)**
- **WALDVOGEL, Siegfried R.**
  **53123 Bonn (DE)**
- **KULISCH, Joerg**
  **53111 Bonn (DE)**
- **MALKOWSKY, Itamar**
  **53121 Bonn (DE)**

(56) Entgegenhaltungen:
**WO-A-2006/005531 DE-A- 2 208 155**

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).

EP 2 041 336 B1

**Beschreibung**

Die vorliegende Erfindung betrifft ein Verfahren zur Herstellung eines Amins durch kathodische Reduktion eines entsprechenden Oximderivates.

Organische Verbindungen mit mehreren chiralen Zentren sind häufig wichtige Bausteine und Auxiliare in der Synthese anderer organischer Verbindungen, um in die letztgenannten chirale Zentren einzuführen oder um bei diesen ein nicht racemisches Stereozentrum aufzubauen. Hierbei werden häufig Verbindungen als Auxiliare gewählt, die ein vergleichsweise starres Grundgerüst aufweisen und zudem voluminöse Gruppen enthalten, die eine dirigierende Wirkung bei der Synthese anderer organischer Verbindungen ausüben können.

Hierbei sind häufig cyclische Verbindungen von Interesse, die aus dem sogenannten "chiral pool" stammen. Dabei sind insbesondere cyclische Terpenoide zu nennen.

Ein solches Beispiel ist 8-Phenylmenthol, das beispielsweise als Auxiliar bei der asymmetrischen Diels-Alder-Reaktion eingesetzt wird (E. Corey, Angew. Chem. 114 (2002), 1724-1741; K.C. Nicolaou, Angew. Chem. 114 (2002), 1742-1773).

Hierbei dient die Hydroxylgruppe des Menthols als funktionelle Gruppe, um das Auxiliar in ein Molekül einzuführen, damit bei einer anschließenden chemischen Reaktion ein prochirales Zentrum in ein chirales Zentrum unter der dirigierenden Wirkung des Auxiliars umgewandelt wird. Hierbei ist jedoch das Menthol, das beispielsweise als Alkoholkomponente eines Esters eingesetzt werden kann, vergleichsweise labil und ist daher nicht für jede Reaktion das Mittel der Wahl.

Es besteht daher ein Bedarf, ein Aminanalogon zu Menthol, dessen Derivaten oder analoger chiraler Auxiliare bereitzustellen.

Das Aminanalogon von Menthol und 8-Methylmenthol wird beispielsweise von M. C. Schopohl et al., Angew. Chem. 115 (2003), 2724-2727 eingesetzt, um die enantiofaciale Differenzierung einzelner Koffein-Gastmoleküle zu untersuchen.

Um zu Aminanaloga des Menthols zu gelangen, schlagen M.C. Schopohl et al., Synthesis 17 (2003), 2689-2694 vor, das entsprechende Amin ausgehend von (+)-Pulegon herzustellen. Als erster Schritt wird bei der Synthese die ethylenische Doppelbindung mit einem Grignard-Reagenz umgesetzt, um einen Rest in 8-Position einzufügen. Dies ermöglicht die Derivatisierung an besagter 8-Position. Aufgrund der Reaktion wird ein chirales Zentrum am Ring-Kohlenstoff in 4-Position erzeugt, wobei die Grignard-Reaktion nicht geeignet ist, zwischen R- und S-Form zu diskriminieren. Hierdurch entsteht ein Epimerengemisch bei der Reaktion. Nach Überführung der Ketofunktion in ein Oxim gelingt die Gewinnung des (4S)-Epimers. Bei der nachfolgenden Beauvault-Blanc-Reaktion mit Natrium in Toluol wird die Oximfunktion in eine Aminofunktion umgewandelt, wobei die Aminofunktion in Bezug auf den Alkylrest in 4-Stellung in Trans-Konfiguration vorliegt.

Dies stellt einen entscheidenden Schritt in der Synthese dar, um eine Konfiguration entsprechend der des Menthols zu erhalten.

Im Gegensatz zu der oben beschriebenen Reduktion mit Natrium ergibt sich bei der heterogenen Reduktion an Übergangsmetallkatalysatoren entweder keine Umsetzung oder das entsprechende Amin wird von der äquimolaren Menge eines epimeren Amins begleitet, das sich nur schwer abtrennen lässt.

Nachteilig an der oben beschriebenen Reaktion ist jedoch der Einsatz des Natriums, das zudem in vergleichsweise großen Mengen eingesetzt wird.

Eine Aufgabe der vorliegenden Erfindung liegt somit darin, ein alternatives Verfahren zur Herstellung eines Amins mit Hilfe eines entsprechenden Oximderivates bereitzustellen, das eine Diskriminierung der R- beziehungsweise S-Form an einem Ring-Kohlenstoff, welches die Oximfunktion oder ein Derivat hiervon trägt und welches in $\alpha$-Position einen Substituenten aufweist, ermöglicht.

Die Aufgabe wird gelöst durch ein Verfahren zur Herstellung eines Amins den Schritt enthaltend

kathodische Reduktion eines entsprechenden Oximderivates der allgemeinen Formel (I)

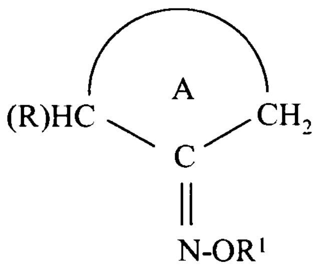

(I),

wobei

R $C_{1-6}$ Alkyl oder $C_{2-6}$ Alkenyl, das gegebenenfalls mit einem oder mehreren Substi- tuenten unabhängig ausgewählt aus der Gruppe bestehend aus Phenyl, O-$C_{1-6}$ Alkyl, NH-$C_{1-6}$ Alkyl, N($C_{1-6}$ Alkyl)$_2$, OH und $NH_2$ substituiert ist;

$R^1$ H; $C_{1-6}$ Alkyl oder C(O)-$C_{1-6}$ Alkyl ist und

A einen 5-, 6- oder 7-gliedrigen Kohlenwasserstoffring darstellt, der gesättigt ist oder eine Doppelbindung aufweist und bei dem gegebenenfalls mindestens eine $CH_2$-Gruppe durch -O-, -S- -NH-, -N=, oder -N($C_{1-6}$ Alkyl)- ersetzt ist und der gegebenenfalls mit einem oder mehreren weiteren Substituenten unabhängig ausgewählt aus der Gruppe bestehend aus Phenyl, $C_{1-6}$ Alkyl, O-$C_{1-6}$ Alkyl, NH-$C_{1-6}$ Alkyl, N($C_{1-6}$ Alkyl)$_2$, OH und $NH_2$ substituiert ist;
und wobei in Bezug auf den Ringkohlenstoff, der den Substituenten R aufweist, das Oximderivat einen Überschuss der R- oder S-Form von mindestens 10 % aufweist.

Es wurde nämlich gefunden, dass überraschenderweise die kathodische Reduktion eines entsprechenden Oximderivates die gewünschte, oben beschriebene Diskriminierung erlaubt und so eine elektrochemische Herstellung von entsprechenden Aminen, bei denen in $\alpha$-Position sich ein Rest R befindet, der zur Aminfunktion in Trans-Position steht, zu ermöglichen, ohne dass beispielsweise elementares Natrium eingesetzt werden müsste.

Bei dem Oximderivat der allgemeinen Formel (I) stellt der Rest R, der in $\alpha$-Position zur späteren Aminfunktion steht, einen $C_{1-6}$-Alkyl- oder $C_{2-6}$-Alkenyl-Rest dar, der unsubstituiert ist oder einen oder mehrere Substituenten aufweist. Als Möglichkeiten für den oder die Substituenten kommen Phenyl, O-$C_{1-6}$-Alkyl, NH-$C_{1-6}$-Alkyl, N($C_{1-6}$-Alkyl)$_2$, OH und $NH_2$ in Frage. Sofern mehrere Substituenten vorhanden sind, können diese unabhängig voneinander aus der oben genannten Gruppe ausgewählt werden.

Vorzugsweise ist der Rest R ein $C_{1-6}$-Alkyl oder $C_{2-6}$-Alkenyl, das unsubstituiert ist oder höchstens einen Substituenten aufweist.

Im Rahmen der vorliegenden Erfindung ist unter dem Begriff "$C_{1-6}$-Alkyl" ein Alkylrest zu verstehen, der 1 bis 6 C-Atome aufweist und unverzweigt oder verzweigt ist. Beispiele solcher Substituenten sind Methyl, Ethyl, n-Propyl, Isopropyl, n-1-Butyl, n-2-Butyl, sec.-Butyl, tert.-Butyl, n-1-Pentyl, n-2-Pentyl, n-3-Pentyl, n-Hexyl.

Weiterhin ist im Rahmen der vorliegenden Erfindung unter dem Begriff "$C_{2-6}$-Alkenyl" ein Alkenylrest zu verstehen, der 2 bis 6 Kohlenstoffatome aufweist und der unverzweigt oder verzweigt ist. Beispiele hierfür sind Vinyl, Allyl, n-1-Propenyl, n-2-Propenyl, Butenyl, Pentenyl, Hexenyl.

Vorzugsweise ist der Rest R ein unsubstituiertes $C_{1-6}$-Alkyl, ein unsubstituiertes $C_{2-6}$-Alkenyl oder ein phenylsubstituiertes $C_{1-6}$-Alkyl.

Ganz besonders bevorzugt ist R Isopropyl, tert.-Butyl oder 1-Phenyl-1-methylethyl.

Bei dem Oximderivat der allgemeinen Formel (I) kann es sich um ein Oxim als solches handeln. Darüber hinaus kann der Wasserstoff der Oximgruppe durch einen $C_{1-6}$-Alkyl- oder C(O)-$C_{1-6}$-Alkyl-Rest ersetzt sein. Vorzugsweise handelt es sich bei dem Oximderivat um ein Oxim ($R^1$ = H) oder $R^1$ ist Methyl oder Acetyl. Ganz besonders bevorzugt handelt es sich bei dem Oximderivat um ein Oxim.

Weiterhin weist das Oximderivat der allgemeinen Formel (I) einen Ring A auf. Dieser beinhaltet den Kohlenstoff, der das Oxim oder ein Analogon des Oxims aufweist, sowie die diesem Kohlenstoff direkt benachbarten Kohlenstoffe. Insgesamt weist der Ring A 5, 6 oder 7 Ringatome auf. Es handelt sich bei dem Ring A um einen Kohlenwasserstoffring, der gesättigt ist oder eine Doppelbindung aufweist. Hierbei kann der Ring A ein Cyclopentan-, Cyclopenten-, Cyclohexan-, Cyclohexen-, Cycloheptan- oder ein Cyclohepten-Ring sein.

Darüber hinaus kann eine $CH_2$-Gruppe des Rings A durch -O-, -S-, -NH- oder -N($C_{1-6}$-Alkyl)- ersetzt sein. Es ist ebenfalls möglich, dass mehrere $CH_2$-Gruppen durch eines der oben genannten Heteroatome ersetzt sind. Für den Fall, dass mehrere Heteroatome als Ringatome im Ring A vorhanden sind, können diese unabhängig voneinander ausgewählt sein. Sofern eine Doppelbindung im Ring A vorhanden ist, kann für den Fall, dass mindestens eine $CH_2$-Gruppe durch ein Heteroatom ausgetauscht ist, und dieses mindestens eine Heteroatom Stickstoff darstellt, dieses als Iminostickstoff auftreten. Es können auch mehrere dieser Stickstoffe vorhanden sein.

Der Ring A kann über den Rest R und die Oximgruppe beziehungsweise deren Analogon hinaus keinen, einen oder mehrere Substituenten aufweisen. Der oder die Substituenten können ausgewählt werden aus der Gruppe bestehend aus Phenyl, -$C_{1-6}$-Alkyl, O-$C_{1-6}$-Alkyl, NH-$C_{1-6}$-Alkyl, N($C_{1-6}$-Alkyl)$_2$, OH und $NH_2$. Sofern mehrere Substituenten vorhanden sind, können diese unabhängig aus der oben genannten Gruppe ausgewählt sein. Vorzugsweise handelt es sich bei dem Substituenten um eine $C_{1-6}$-Alkylgruppe. Insbesondere bevorzugt ist Methyl.

Der Ring A weist vorzugsweise genau einen weiteren Substituenten auf.

Selbstverständlich weist der Ring A des Oximderivates der allgemeinen Formel (I) in jedem Falle den Rest R sowie das Oxim beziehungsweise das Oximanalogon als Substituenten auf.

Sofern der Ring A nicht ausschließlich aus Kohlenstoffatomen gebildet wird, kann der Ring beispielsweise ein Tetrahydrofuran, Dihydrothiophen, Dihydrofuran, Dihydropyrrol, Oxan, Thian, Piperidin, Dihydropyran oder Tetrahydropyridin sein. Vorzugsweise weist der Ring A jedoch keine Heteroatome auf. Ganz besonders bevorzugt handelt es sich bei dem Ring A um Cyclohexan oder Cyclohexen, insbesondere Cyclohexan.

Ganz besonders bevorzugt handelt es sich bei dem Oximderivat der allgemeinen Formel (I) um ein methylsubstituiertes Oximderivat ausgewählt aus der Gruppe bestehend aus

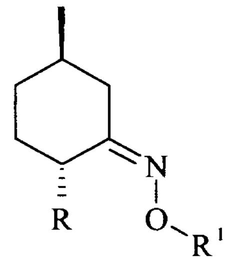 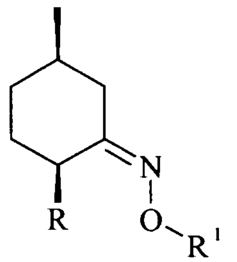 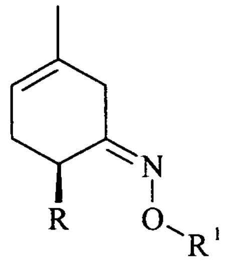 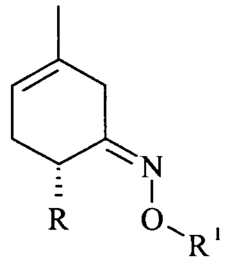.

Ganz besonders bevorzugt sind Oximderivate der allgemeinen Formel (I), die nach der Reduktion Menthylamin, 8-Methylmenthylamin oder 8-Phenylmenthylamin liefern.

Hierbei haben die Reste R, $R^1$ die oben bezeichnete Bedeutung.

Die Oximderivate für das erfindungsgemäße Verfahren sind über im Stand der Technik bekannte Herstellverfahren zugänglich. Hierbei ist typischerweise eine Vorstufe das entsprechende Keton.

Eine Möglichkeit zur Herstellung besteht darin, von dem entsprechenden aromatischen System auszugehen, das die gewünschten Substituenten trägt, um nach Reduktion und Diastereomerentrennung das gewünschte Oximderivat aufzubauen. Sofern im Ring A eine Doppelbindung vorhanden ist, könnte diese durch stufenweise Reduktion oder über eine Reduktions-/Eliminierungsreaktion erhalten werden. Trennungen sind beispielsweise von H. Feltkamp et al., Liebigs Ann. Chem. 707 (1967), 78-86 beschrieben.

Vorzugsweise erfolgt die Synthese zum Oxim unter Verwendung des sogenannten "Chiral Pools". Eine solche Strategie wird beispielsweise von M.C. Schopohl et al., Synthesis 17 (2003), 2689-2694 beschrieben. Ein bevorzugtes Ausgangsmaterial ist Pulegon oder Menthon.

Ziel der vorliegenden Erfindung ist es, ein Verfahren zur kathodischen Reduktion eines Oximderivates zu einem entsprechenden Amin bereitzustellen, bei dem die Oximfunktion oder das Oximanalogon in $\alpha$-Position einen Rest R aufweist, wobei nach Reduktion die Aminofunktion zum Rest R überwiegend in Trans-Position steht. Dies setzt jedoch voraus, dass das chirale Ring-C-Atom, welches den Rest R aufweist, nicht in racemischer Form vorliegt.

Es soll daher im Rahmen der vorliegenden Erfindung ein Überschuss der R- oder S-Form von mindestens 10 % im Oximderivat der allgemeinen Formel (I) vorliegen, vorzugsweise mindestens 50 %, weiterhin bevorzugt mindestens 75 %, weiterhin mehr bevorzugt mindestens 90 %, weiterhin mehr bevorzugt mindestens 95 %, weiterhin mehr bevorzugt mindestens 98 %, weiterhin mehr bevorzugt mindestens 99 %, insbesondere mindestens 99,9 %. Ganz besonders bevorzugt liegt der Ringkohlenstoff, der den Substituenten R aufweist, ausschließlich in der R- oder S-Form vor.

Sind darüber hinaus in dem Oximderivat weitere Stereozentren vorhanden, gilt für diese das Analoge.

Der Überschuss der R- oder S-Form ergibt sich aus der Formel

$$E[\%] = \frac{|n_R - n_S|}{n_R + n_S} \bullet 100,$$

wobei E den Überschuss in % bezeichnet und n jeweils die Stoffmenge der R- oder S-Form in Bezug auf den Ringkohlenstoff, der den Substituenten R trägt, bedeutet.

Sofern der Ringkohlenstoff, der den Substituenten R aufweist, das einzige Stereozentrum im Oximderivat der allgemeinen Formel (I) ist, handelt es sich um den Enantiomerenüberschuss des Oximderivats.

Sofern darüber hinaus mindestens ein weiteres Stereozentrum vorhanden ist, handelt es sich bei dem Überschuss um einen Epimerenüberschuss in Bezug auf den Ringkohlenstoff, der den Substituenten R aufweist.

Die Bestimmung der obigen Größen zur Ermittlung des Überschusses ist mit Hilfe von im Stand der Technik bekannten Methoden möglich. Sofern es sich um einen Enantiomerenüberschuss handelt, kann eine entsprechende Auswertung über gängige Methoden wie beispielsweise Circulardichroismus erfolgen. Eine weitere Methode zur Ermittlung wäre durch eine Derivatisierung des Oximderivates der allgemeinen Formel (I) gegeben, so dass das erhaltene

Produkt als Diastereomer vorliegt und die gängigen Methoden für Diastereomere angewendet werden. Eine der gängigsten Methoden ist die Kernresonanzspektroskopie.

Nach Reduktion des Oxims zum Amin liegt ein weiteres chirales Kohlenstoffatom vor, wobei aufgrund der elektrochemischen Reduktion eine Diskriminierung zwischen Rund S-Form erfolgt, so dass ein Überschuss in Bezug auf dieses Kohlenstoffatom erzielt wird, der größer 0 % ist. Vorzugsweise beträgt der Überschuss mindestens 10 %, mehr bevorzugt mindestens 50 %, insbesondere mindestens 60 %.

Die elektrochemische Reduktion von Oximen an einer Kathode ist aus WO-A 2006/005531 bekannt.

Die Reduktion kann beispielsweise in einer geteilten oder ungeteilten Durchflusszelle erfolgen. Vorzugsweise findet eine geteilte Durchflusszelle Anwendung.

Gegebenenfalls enthält der Katholyt neben dem im Laufe der Reaktion gebildeten Amin und dem Oximderivat ein Lösungsmittel. Dabei handelt es sich um die in der organischen Chemie allgemein üblichen inerten Lösungsmittel wie Dimethylcarbonat, Propylencarbonat, Tetrahydrofuran, Dimethoxyethan, Acetonitril oder Dimethylformamid. Bevorzugt wird als Lösungsmittel ein $C_1$-$C_4$-Alkylalkohol eingesetzt. In Kombination mit den genannten Lösungsmitteln eignen sich auch $C_5$-$C_7$-Kohlenwasserstoffe wie z.B. Hexan als Lösungsmittel. Ebenso sind cyclische oder acyclische Ether einsetzbar. Ebenso kann Wasser eingesetzt werden. Zudem können Mischungen der oben genannten Lösemittel eingesetzt werden.

Bevorzugt sind Alkohole, Wasser, Ether oder Mischungen davon. Hierbei kann sich ein ein- oder mehrphasiges System ausbilden.

Ganz besonders bevorzugt sind Alkohole, die gegebenenfalls in Mischungen miteinander und/oder Wasser vorliegen und $C_{1-4}$-Alkanole, wie Methanol, Ethanol, n-Propanol, i-Propanol, n-Butanol, sec-Butanol, tert.-Butanol; Diole wie Glykol, Triole wie Glycerin, Polyole, Polyether oder Alkoxyalkanole wie Ethylenglykolmonomethylether oder Dimethoxyethan. Ganz besonders bevorzugt ist Dimethoxyethan.

Zur Herstellung der Leitfähigkeit enthält der Katholyt im Allgemeinen eine Mineralsäure, bevorzugt Schwefelsäure oder ein Alkali($C_1$-bis $C_4$)-alkylalkoholat, bevorzugt Natrium-Methanolat.

Im Allgemeinen setzt man dem Anolyt und ggf. auch dem Katholyt (zusätzlich zu einem der oben genannten die Leitfähigkeit herstellenden Mittel) ein Leitsalz zu. Dabei handelt es sich im Allgemeinen um Alkali, Tetra($C_1$-$C_6$-alkyl) ammonium-, bevorzugt Tri($C_1$-$C_6$-alkyl)-methylammoniumsalze. Als Gegenion kommen Sulfat, Hydrogensulfat, Alkylsulfate, Arylsulfate, Halogenide, Phosphate, Carbonate, Alkylphosphate, Alkylcarbonate, Nitrat, Alkoholate, Tetrafluoroborat, Hexafluorophosphat oder Perchlorat in Betracht.

Bevorzugt sind Methyltributylammoniummethylsulfate (MTBS), Methyltriethylammoniummethylsulfat oder Methyl-tri-propylammoniummethylsulfate.

Der Anolyt enthält vorzugsweise ebenfalls ein Lösemittel, das Wasser, einen Alkohol oder mehrere Alkohole oder eine Mischung von zwei oder mehreren der Lösemittel enthält. Prinzipiell kommen für den Anolyten die gleichen Alkohole in Betracht wie für den Katholyt, diese können jedoch auch verschieden zum Katholyten sein.

Das erfindungsgemäße Verfahren kann vorzugsweise in allen üblichen geteilten Elektrolysezellentypen durchgeführt werden, um im Rahmen des erfindungsgemäßen Verfahrens ausschließen zu können, dass Edukte wie Produkte durch den Kathodenprozess chemische Nebenreaktionen unterlaufen. Vorzugsweise arbeitet man kontinuierlich in geteilten Durchflusszellen.

Geteilte Zellen mit planparalleler Elektrodenanordnung kommen bevorzugt zum Einsatz. Als Trennmedien können Ionenaustauschermembranen, mikroporöse Membranen, Diaphragmen, Filtergewebe aus nicht elektronenleitenden Materialien, Glasfritten, sowie poröse Keramiken eingesetzt werden. Vorzugsweise werden Ionenaustauschermembranen, insbesondere Kationaustauschermembranen verwendet. Diese leitfähigen Membranen sind handelsüblich z.B. unter dem Handelsnamen Nafion® (Fa. E.T. DuPont de Nemours and Company) und Gore Select® (Fa. W. L. Gore & Associates, Inc.) erhältlich.

Als Kathoden verwendet man bevorzugt solche, bei denen die Kathodenoberfläche gebildet ist aus einem Material mit hoher Wasserstoffüberspannung, z.B. aus Blei, Zink, Zinn, Nickel, Quecksilber, Cadmium, Kupfer oder Legierungen dieser Metalle oder Glassy Cabon, Grafit oder Diamant.

Besonders bevorzugt sind Diamantelektroden, wie z.B. in der EP-A-1036863 beschrieben sind.

Als Anoden kommen grundsätzlich alle üblichen Materialien in Betracht, bevorzugt die, die auch als Kathodenmaterialien genannt. Im sauren Anolyten werden bevorzugt Platin-, Diamant-, Glassy Carbon- oder Grafitanoden oder die dem Fachmann bekannte dimensionsstabile Anoden (DSA) eingesetzt. Ist der Anolyt basisch, wird bevorzugt Edelstahl verwendet.

Die Anodenreaktion kann frei gewählt werden, bevorzugt wird das Lösungsmittel, beispielsweise ein $C_1$-$C_4$-Alkohol oxidiert. Bei der Verwendung von Methanol wird Methylformiat, Formaldehyd-Dimethylacetal bzw. Dimethylcarbonat gebildet. Hierzu dient z.B. eine mit einem $C_1$-$C_4$-Alkohol verdünnte Schwefelsäurelösung.

Die Stromdichten, bei denen man das Verfahren durchführt, betragen im allgemeinen 1 bis 1000, bevorzugt 10 bis 100 $mA/cm^2$. Im Allgemeinen wird bei Normaldruck gearbeitet. Höhere Drücke werden bevorzugt dann angewandt, wenn bei höheren Temperaturen gearbeitet werden soll, um eine Sieden der Ausgangsverbindungen bzw. Lösungsmittel

zu vermeiden.

Nach Beendigung der Reaktion wird die Elektrolytlösung nach allgemeinen Trennmethoden aufgearbeitet. Hierzu wird der Katholyt im Allgemeinen zunächst destilliert und die einzelnen Verbindungen in Form von unterschiedlichen Fraktionen getrennt gewonnen. Eine weitere Reinigung kann beispielsweise durch Kristallisation, Destillation oder chromatographisch erfolgen.

**Beispiele**

**Beispiel 1**

Elektrochemische Reduktion von (-)-Menthonoxim

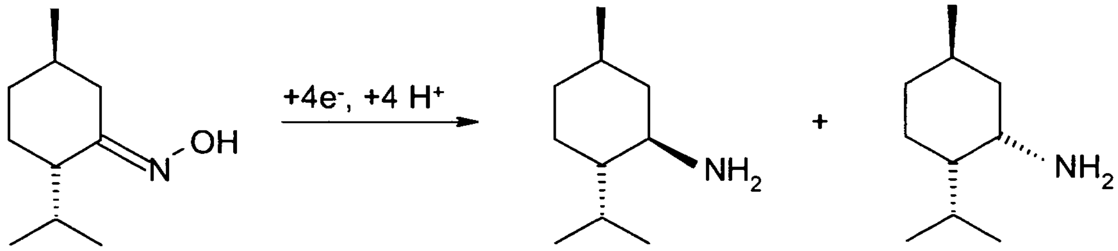


1 2 3

In einer geteilten Elektrolysezelle werden Anolyt und Katholyt nach den unten angegebenen Varianten bei einer Stromdichte von 67 mA/cm² elektrochemisch umgesetzt, wobei 8 F/mol eingespeist werden (entspricht 2 Äquivalenten). Die Elektrolyse wird bei Raumtemperatur durchgeführt; die geteilte Zelle wird aus zwei Flanschzellen konstruiert, wobei Anoden- und Kathodenhalbraum durch eine Nafion-324-Membran getrennt werden. Anodenmaterial ist Platin, als Kathode wird Blei verwendet.

Zur Aufarbeitung wird das Reaktionsgemisch mit Wasser (50 mL) verdünnt, mit konzentrierter Schwefelsäure auf pH = 1 eingestellt und mit Cyclohexan gewaschen (4 x 30 mL). Die wässrige Phase wird anschließend mit 50 %iger Kalilauge auf pH = 12 gebracht und erneut mit TBME extrahiert (4 x 50 mL). Die vereinigten organischen basischen Extrakte werden mit 10 %iger Natronlauge (2 x 100 mL) und über Calciumoxid getrocknet und am Rotationsverdampfer vom Lösungsmittel befreit. Eine weitere Aufreinigung kann zum Beispiel durch Säulenchromatographie oder Destillation erfolgen.

**Variante A: Selektiv für 2**

| | |
|---|---|
| Anolyt: | 20 mL 1 M $H_2SO_4$ |
| | 20 mL Methanol |
| Katholyt: | 2.05 g (-)-Menthonoxim (1) (12,1 mmol) |
| | 20 mL 1 M $H_2SO_4$ |
| | 20 mL DME |
| Kathodenmaterial: | Quecksilber |
| Stromdichte: | 23.3 mA/cm² |
| Temperatur: | -14 - - 18 °C |
| Q/n: | 6.6 F/mol **1** (entspricht 1,65 Äquivalenten) |
| Ausbeute: | 56 % (1,06 g, 6,8 mmol); **2**:**3** = 4:1 (Epimerenverhältnis aus chromatographisch Untersuchungen der quantitativ zu Acetamiden umgesetzten Produkte bestimmt (Epimerenüberschuss de = 60%) |

**Variante B: Selektiv für 3**

| | |
|---|---|
| Anolyt: | 50 mL 1 M $H_2SO_4$<br>50 mL Methanol |
| Katholyt: | 2.26 g (-)-Menthonoxim (1) (13 mmol)<br>25 mL 1 M $H_2SO_4$<br>75 mL Ethylenglykolmonomethylether |
| Kathodenmaterial: | Blei |
| Stromdichte: | 50 mA/cm$^2$ |
| Q/n: | 6 F/mol **1** (entspricht 1,5 Äquivalenten) |
| Ausbeute: | 13 % (0,93 g, 6 mmol); 2:3 = 1 : 0.57 (Epimerenverhältnis aus chromatographisch Untersuchungen der zu Acetamiden umgesetzten Produkte bestimmt) |

**Variante C: Epimerengemisch**

| | |
|---|---|
| Anolyt: | 40 mL 1 M $H_2SO_4$ |
| Katholyt: | 3.26 g (-)-Methonoxim (1) (21,7 mmol)<br>20 mL 1 M $H_2SO_4$<br>20 mL 1,4-Dioxan |
| Kathodenmaterial: | Blei |
| Ausbeute: | 50 % (1,69 g, 10,9 mmol); 2:3 = 5:4 (Epimerenverhältnis durch NMR bestimmt) (de = 11 %) |

**Beispiel 2**

Elektrochemische Reduktion von (-)-(*E*)-(1*R*,4*S*)-8-Phenylmenthyloxim Verwendung von Leitsalzen als Additiv

| | |
|---|---|
| Anolyt: | 50 mL 0.162 M $H_2SO_4$ in Methanol (entspricht 2 Gew.-%). |
| Katholyt: | 0.51 g (-)-(*E*)-(1*R*,4*S*)-8-Phenylmenthyloxim (0.002 mol)<br>50 mL 0.162 M $H_2SO_4$,<br>0.017 M Triethylmethylammoniummethylsulfat in Methanol (entspricht 2 Gew.-%, 0,5 Gew.-%) |
| Kathodenmaterial: | Blei |
| Stromdichte: | 12.5 mA/cm$^2$ |
| Q/n: | 10 F/mol (-)-(*E*)-(1*R*,4*S*)-8-Phenylmenthyloxim (entspricht 2.5 Äquivalenten) |
| Temperatur: | 20 - 22°C |
| Ausbeute: | 94 % (0.45 g, 0.002 mol); Diastereomerenverhältnis (-)-(1*R*,3*R*,4*S*)-8-Phenylmenthylamin : (+)-(1*R*,3*R*,4*R*)-8-Phenylmenthylamin **6.5** : **1** (bestimmt aus $^1$H-NMR-spektroskopischen Untersuchungen), de = 73 % |
| Elektrolyseapparatur: | geteilte Elektrolysezelle |
| Trennmedium: | Nafion® |

Durchführung:

Das (-)-(*E*)-(1*R*,4*S*)-8-Phenylmenthyloxim wird im Katholyt gelöst und in die Kathodenhalbzelle überführt. Die Anodenhalbzelle wird mit dem Anolyten befüllt. Die Elektrolyse erfolgt galvanostatisch bei einer Stromdichte von 12.5 mA/cm$^2$ und einer Temperatur von 20-22°C. Als Anode dient Platin, als Kathode Blei. Nach einer übergegangenen Ladungsmenge von 1966 C (10 F/mol (-)-(*E*)-(1*R*,4*S*)-8-Phenylmenthyloxim, 2.5 Äquivalenten) wird die Elektrolyse beendet.

Aufarbeitung:

Das Reaktionsgemisch wird aus der Kathodenhalbzelle in einen Kolben überführt und die Zelle mit insgesamt 50 mL Methanol und 50 mL Wasser gespült. Der gesamte Kathodenaustrag wird mit konzentrierter Schwefelsäure auf pH = 1 eingestellt und unter vermindertem Druck von Methanol befreit. Es wird mit Cyclohexan gewaschen (4 × 30 mL) und der pH-Wert der wässrige Phase im Folgenden mit 50%iger Kalilauge auf pH = 12 eingestellt. Es wird mit TBME extrahiert (4 × 50 mL), die vereinigte organische Phase über Calciumoxid getrocknet und am Rotationsverdampfer von Lösungsmittel befreit. Das (1*R*,3*R*)-8-Phenylmenthylamin wird als leicht gelbe Flüssigkeit erhalten.

Beobachtungen:

Die Klemmspannung während der Elektrolyse liegt hoch (bis zu 20.6 V). Nach einiger Zeit bildet sich an der Kathode ein Belag aus. Wohingegen zu Anfang nur eine geringe Gasentwicklung an der Kathode zu beobachten ist, wird diese mit zunehmender Belagbildung immer stärker.

Analytische Daten:

(-)-(1*R*,3*R*,4*S*)-8-Phenylmenthylamin

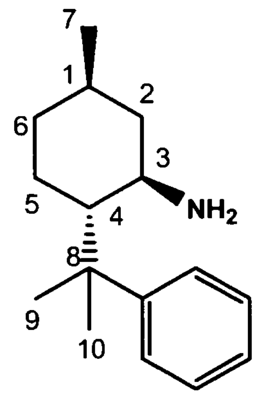

$t_R^A$ = 20.20 min
$^1$H-NMR (400 MHz; $CDCl_3$)
δ (ppm) = 0.75 (m, 1*H*, 2-$H_a$); 0.87 (d, 3H, 7-H, $^3J_{7,1}$ = 6.5 Hz); 0.91 - 0.95 (m, 1H, 5-$H_a$); 1.06 - 1.16 (m, 1H, 6-$H_a$); 1.22 (s, 3H, 10-*H*); 1.38 (s, 3H, 9-*H*); 1.40 - 1.41 (m, 1H, 1-$H_a$); 1.62 - 1.73 (m, 3H, 2-$H_e$, 4-$H_a$, 6-$H_e$); 1.79 - 1.85 (m, 1H, 5-$H_e$); 2.60 - 2.66 (m, 1H, 3-$H_a$); 7.13 - 7.17, 7.28 - 7.32, 7.37 - 7.40 (m, 5H, Ar-*H*).
$^{13}$C-NMR (100 MHz; $CDCl_3$)
δ (ppm) = 22.2 (C-7); 22.7 (C-9); 27.0 (C-5); 30.6 (C-10); 32.0 (C-1); 35.3 (C-6); 39.9 (C-8); 46.7 (C-2); 53.2 (C-3); 54.2 (C-4); 125.3 (CH-Ar); 128.3 (2×CH-Ar); 152.9 ($C_q$-Ar).
$^A$: Gerät: GC2010 Fa. *Shimadzu,* Japan.
Säule: Quarzkappilaresäule HP-5 Fa. *Agilent,* USA (Länge 30 m; Innendurchmesser: 0.25 mm; Filmdicke der kovalent gebundenen stationären Phase: 0.25 µm; Trägergas: Wasserstoff; Injektortemperatur: 250°C; Detektortemperatur: 300°C; Säulenvordruck: 106 kPa).
Programm: 50°(10°-80)(2°-100)(15°-270)5'.

## Patentansprüche

1. Verfahren zur Herstellung eines Amins den Schritt enthaltend
kathodische Reduktion eines entsprechenden Oximderivates der allgemeinen Formel (I)

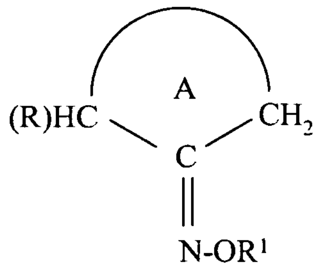

(I),

wobei
R $C_{1-6}$ Alkyl oder $C_{2-6}$ Alkenyl, das gegebenenfalls mit einem oder mehreren Substituenten unabhängig ausgewählt aus der Gruppe bestehend aus Phenyl, O-$C_{1-6}$ Alkyl, NH-$C_{1-6}$ Alkyl, N($C_{1-6}$ Alkyl)$_2$, OH und $NH_2$ substituiert ist;
$R^1$ H; $C_{1-6}$ Alkyl oder C(O)-$C_{1-6}$ Alkyl ist und
A einen 5-, 6- oder 7-gliedrigen Kohlenwasserstoffring darstellt, der gesättigt ist oder eine Doppelbindung aufweist und bei dem gegebenenfalls mindestens eine $CH_2$-Gruppe durch -O-, -S- -NH-, -N=, oder -N($C_{1-6}$ Alkyl)- ersetzt ist und der gegebenenfalls mit einem oder mehreren weiteren Substituenten unabhängig ausgewählt aus der Gruppe bestehend aus Phenyl, $C_{1-6}$ Alkyl, O-$C_{1-6}$ Alkyl, NH-$C_{1-6}$-Alkyl, N($C_{1-6}$ Alkyl)$_2$, OH und $NH_2$ substituiert ist;
und wobei in Bezug auf den Ringkohlenstoff, der den Substituenten R aufweist, das Oximderivat einen Überschuss der R- oder S-Form von mindestens 10 % aufweist.

**2.** Verfahren nach Anspruch 1, **dadurch gekennzeichnet, dass** R Isopropyl, tert.-Butyl oder 2-Phenyl-2-propyl ist.

**3.** Verfahren nach Anspruch 1 oder 2, **dadurch gekennzeichnet, dass** $R^1$ Wasserstoff ist.

**4.** Verfahren nach einem der Ansprüche 1 bis 3, **dadurch gekennzeichnet, dass** A ein Cyclohexan oder Cyclohexen ist, das gegebenenfalls mit einem oder mehreren Methylgruppen substituiert ist.

**5.** Verfahren nach einem der Ansprüche 1 bis 4, **dadurch gekennzeichnet, dass** in Bezug auf den Ringkohlenstoff, der den Substituenten R aufweist, das Oximderivat einen Epimerenüberschuss von mindestens 98 % aufweist.

**6.** Verfahren nach einem der Ansprüche 1 bis 5, **dadurch gekennzeichnet, dass** das Oximderivat ausgewählt ist aus der Gruppe bestehend aus

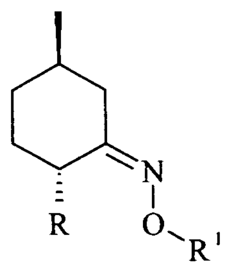

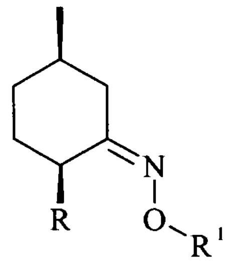

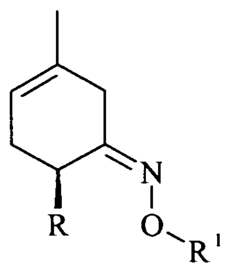

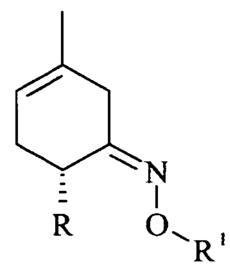

**7.** Verfahren nach einem der Ansprüche 1 bis 6, **dadurch gekennzeichnet, dass** das erhaltene Produkt Menthylamin, 8-Methylmenthylamin oder 8-Phenylmenthylamin ist.

**8.** Verfahren nach einem der Ansprüche 1 bis 7, **dadurch gekennzeichnet, dass** die Reduktion in einer geteilten Durchflusszelle erfolgt.

**9.** Verfahren nach einem der Ansprüche 1 bis 8, **dadurch gekennzeichnet, dass** der Katolyt Wasser, mindestens einen Alkohol oder Ether oder Mischungen davon enthält.

**10.** Verfahren nach einem der Ansprüche 1 bis 9, **dadurch gekennzeichnet, dass** der Anolyt Wasser, mindestens ein Alkohol oder eine Mischung davon enthält.

## Claims

1. A process for preparing an amine, which comprises the step
cathodic reduction of a corresponding oxime derivative of the general formula (I)

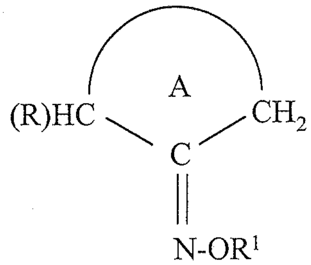

(I),

where
R is $C_{1-6}$-alkyl or $C_{2-6}$-alkenyl which is optionally substituted by one or more substituents selected independently from the group consisting of phenyl, O-$C_{1-6}$-alkyl, NH-$C_{1-6}$-alkyl, N($C_{1-6}$-alkyl)$_2$, OH and $NH_2$;
$R^1$ is H; $C_{1-6}$-alkyl or C(O)-$C_{1-6}$-alkyl and
A is a 5-, 6- or 7-membered hydrocarbon ring which is saturated or has a double bond and in which at least one $CH_2$ group may, if appropriate, be replaced by -O-, -S-, -NH-, -N= or -N($C_{1-6}$-alkyl)- and which may optionally be substituted by one or more further substituents selected independently from the group consisting of phenyl, $C_{1-6}$-alkyl, O-$C_{1-6}$-alkyl, NH-$C_{1-6}$-alkyl, N($C_{1-6}$-alkyl)$_2$, OH and $NH_2$;
and, based on the ring carbon bearing the substituent R, the oxime derivative has an excess of the R or S form of at least 10%.

2. The process according to claim 1, wherein R is isopropyl, tert-butyl or 2-phenyl-2-propyl.

3. The process according to claim 1 or 2, wherein $R^1$ is hydrogen.

4. The process according to any of claims 1 to 3, wherein A is a cyclohexane or cyclohexene which may optionally be substituted by one or more methyl groups.

5. The process according to any of claims 1 to 4, wherein, based on the ring carbon bearing the substituent R, the oxime derivative has an epimeric excess of at least 98%.

6. The process according to any of claims 1 to 5, wherein the oxime derivative is selected from the group consisting of

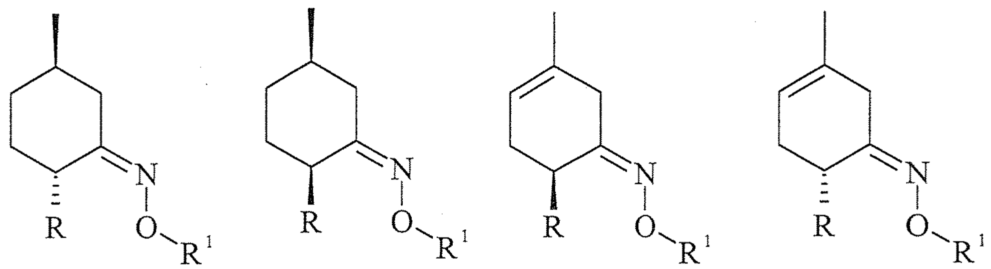

7. The process according to any of claims 1 to 6, wherein the product obtained is menthylamine, 8-methylmenthylamine or 8-phenylmenthylamine.

8. The process according to any of claims 1 to 7, wherein the reduction is carried out in a divided flow cell.

9. The process according to any of claims 1 to 8, wherein the catholyte comprises water, at least one alcohol or ether

or mixtures thereof.

**10.** The process according to any of claims 1 to 9, wherein the anolyte comprises water, at least one alcohol or a mixture thereof.

**Revendications**

**1.** Procédé de fabrication d'une amine, comprenant l'étape
réduction cathodique d'un dérivé d'oxime correspondant de formule générale (I)

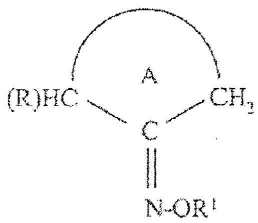
(I),

dans laquelle
R représente un alkyle en $C_{1-6}$ ou un alcényle en $C_{2-6}$, qui est éventuellement substitué par un ou plusieurs substituants choisis indépendamment dans le groupe constitué par phényle, O-alkyle en $C_{1-6}$, NH-alkyle en $C_{1-6}$, N(alkyle en $C_{1-6}$)$_2$, OH et $NH_2$ ;
$R^1$ représente H ; un alkyle en $C_{1-6}$ ou un C(O)-alkyle en $C_{1-6}$ et
A représente un cycle hydrogénocarboné à 5, 6 ou 7 éléments, qui est saturé ou comporte une double liaison et dans lequel éventuellement au moins un groupe $CH_2$ est remplacé par -O-, -S-, -NH-, -N= ou- N(alkyle en $C_{1-6}$ )- et qui est éventuellement substitué par un ou plusieurs substituants supplémentaires choisis indépendamment dans le groupe constitué par phényle, alkyle en $C_{1-6}$, O-alkyle en $C_{1-6}$, NH-alkyle en $C_{1-6}$, N(alkyle en $C_{1-6}$)$_2$, OH et $NH_2$ ;
et le dérivé d'oxime comprenant, au regard du carbone cyclique qui porte le substituant R, un excès de la forme R ou S d'au moins 10 %.

**2.** Procédé selon la revendication 1, **caractérisé en ce que** R est isopropyle, tert.-butyle ou 2-phényl-2-propyle.

**3.** Procédé selon la revendication 1 ou 2, **caractérisé en ce que** $R^1$ est l'hydrogène.

**4.** Procédé selon l'une quelconque des revendications 1 à 3, **caractérisé en ce que** A est un cyclohexane ou cyclohexène, qui est éventuellement substitué par un ou plusieurs groupes méthyle.

**5.** Procédé selon l'une quelconque des revendications 1 à 4, **caractérisé en ce que** le dérivé d'oxime comprend, au regard du carbone cyclique qui porte le substituant R, un excès d'épimères d'au moins 98 %.

**6.** Procédé selon l'une quelconque des revendications 1 à 5, **caractérisé en ce que** le dérivé d'oxime est choisi dans le groupe constitué par

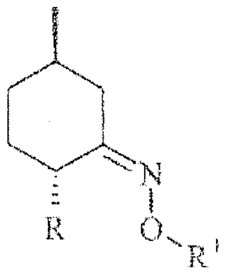 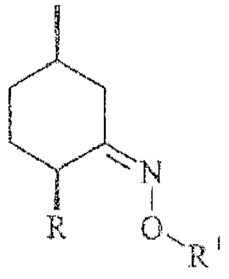 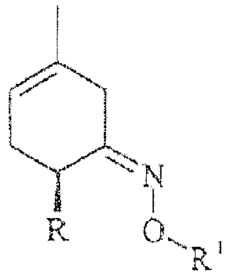 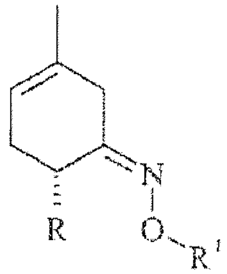

**7.** Procédé selon l'une quelconque des revendications 1 à 6, **caractérisé en ce que** le produit obtenu est la menthylamine, la 8-méthylmenthylamine ou la 8-phénylmenthylamine.

**8.** Procédé selon l'une quelconque des revendications 1 à 7, **caractérisé en ce que** la réduction a lieu dans une cellule à flux continu partagée.

**9.** Procédé selon l'une quelconque des revendications 1 à 8, **caractérisé en ce que** le catholyte contient de l'eau, au moins un alcool ou un éther ou leurs mélanges.

**10.** Procédé selon l'une quelconque des revendications 1 à 9, **caractérisé en ce que** l'anolyte contient de l'eau, au moins un alcool ou un de leurs mélanges.

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente


- WO 2006005531 A
- EP 1036863 A


### In der Beschreibung aufgeführte Nicht-Patentliteratur


- **E. Corey.** *Angew. Chem.,* 2002, vol. 114, 1724-1741
- **K.C. Nicolaou.** *Angew. Chem.,* 2002, vol. 114, 1742-1773
- **von M. C. Schopohl et al.** *Angew. Chem.,* 2003, vol. 115, 2724-2727
- **M.C. Schopohl et al.** *Synthesis,* 2003, vol. 17, 2689-2694
- **von H. Feltkamp et al.** *Liebigs Ann. Chem.,* 1967, vol. 707, 78-86
- **von M.C. Schopohl et al.** *Synthesis,* 2003, vol. 17, 2689-2694